(19) Europäisches Patentamt
European Patent Office
Office européen des brevets

(11) Publication number : **0 560 483 A1**

# (12) EUROPEAN PATENT APPLICATION

(21) Application number : **93300817.9**

(51) Int. Cl.[5] : **C07D 261/08,** A01N 43/80

(22) Date of filing : **04.02.93**

(30) Priority : **12.03.92 US 850035**

(43) Date of publication of application : **15.09.93 Bulletin 93/37**

(84) Designated Contracting States : **AT BE CH DE DK ES FR GB GR IE IT LI LU NL PT SE**

(71) Applicant : **RHONE-POULENC AGRICULTURE LTD.**
**Fyfield Road**
**Ongar, Essex CM5 0HW (GB)**

(72) Inventor : **Cain, Paul Alfred**
**c/o Rhone-Poulenc Agriculture Ltd, Fyfield Road**
**Ongar, Essex CM5 0HW (GB)**
Inventor : **Cramp, Susan Mary**
**c/o Rhone-Poulenc Agriculture Ltd, Fyfield Road**
**Ongar, Essex CM5 0HW (GB)**

(74) Representative : **Bentham, Stephen**
**J.A. Kemp & Co. 14 South Square Gray's Inn**
**London, WC1R 5LX (GB)**

## (54) 4 Benzopyl isoxazole derivatives and their use as herbicides.

(57) 4-Benzoylisoxazole derivative of general formula (I) :

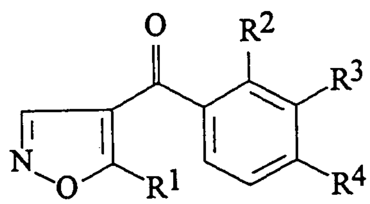

(I)

wherein :

$R^1$ represents an alkyl group or an optionally substituted cycloalkyl group

$R^2$ represents a halogen atom, or a group selected from $R^5$, $-OR^5$, $-S(O)_mR^5$, $-O(CH_2)_q-OR^5$, $-CO_2R^5$ and nitro ;

$R^3$ represents a halogen atom, or a group selected from $R^5$, $-OR^5$, $-S(O)_mR^5$ and $-O(CH_2)_q-OR^5$ ;

$R^4$ represents a hydrogen or halogen atom, or a group selected from $R^5$, $-OR^5$, $-S(O)_mR^5$, $-O(CH_2)_q-OR^5$ and nitro ;

with the proviso that at least one of the groups $R^2$, $R^3$ and $R^4$ represents $-O(CH_2)_q-OR^5$ ;

$R^5$ represents a straight- or branched- chain alkyl group containing from one to four carbon atoms optionally substituted by one or more halogen atoms ;

m represents zero, one or two ; and

q represents an integer from one to three ;.

and their use as herbicides is described.

EP 0 560 483 A1

This invention relates to novel 4-benzoylisoxazole derivatives, compositions containing them and their use as herbicides. Herbicidal 4-benzoylisoxazoles are described in European Patent Publication Number 0418175.

The present invention provides 4-benzoylisoxazole derivatives of general formula (I)

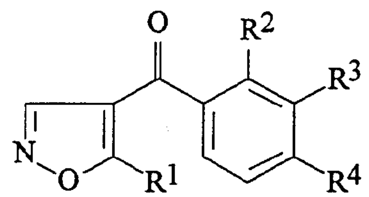

(I)

wherein:

$R^1$ represents a straight- or branched- chain alkyl group containing from one to four carbon atoms or a three or four membered cycloalkyl group which is optionally substituted by a straight- or branched- chain alkyl group containing up to four carbon atoms;

$R^2$ represents a halogen atom, or a group selected from $R^5$, $-OR^5$, $-S(O)_mR^5$, $-O(CH_2)_q-OR^5$, $-CO_2R^5$ and nitro;

$R^3$ represents a halogen atom, or a group selected from $R^5$, $-OR^5$, $-S(O)_mR^5$ and $-O(CH_2)_q-OR^5$;

$R^4$ represents a hydrogen or halogen atom, or a group selected from $R^5$, $-OR^5$, $-S(O)_mR^5$, $-O(CH_2)_q-OR^5$ and nitro;

with the proviso that at least one of the groups $R^2$, $R^3$ and $R^4$ represents $-O(CH_2)_q-OR^5$;

$R^5$ represents a straight- or branched- chain alkyl group containing from one to four carbon atoms optionally substituted by one or more halogen atoms;

m represents zero, one or two; and

q represents an integer from one to three.

The compounds of the invention show unexpected and high herbicidal action in comparison with known compounds against important weed species including cockelbur (Xanthium strumarium).

Compounds of formula (I) in which $R^3$ represents $-O(CH_2)_q-OR^5$ are preferred.

A preferred class of compounds of formula (I) because of their herbicidal properties are those wherein:

$R^2$ represents a halogen atom or a group selected from $R^5$, $-OR^5$, $-S(O)_mR^5$, $-CO_2R^5$ and nitro;

$R^3$ represents $-O(CH_2)_q-OR^5$;

$R^4$ represents a hydrogen or halogen atom, or a group selected from $R^5$, $-OR^5$, $-S(O)_mR^5$ and nitro; and

q represents two or three.

A further preferred class of compounds of formula (I) because of their herbicidal properties are those having one or more of the following features:

$R^1$ represents a methyl, ethyl, 1-methylethyl, cyclopropyl or 1-methylcyclopropyl group;

$R^2$ represents a chlorine, bromine or fluorine atom, or a group selected from $R^5$ and $-S(O)_mR^5$;

$R^4$ represents a chlorine, bromine or fluorine atom, or a group selected from $R^5$ and $-S(O)_mR^5$;

$R^5$ represents a straight- or branched- chain alkyl group containing from one to four carbon atoms optionally substituted by one or more halogen atoms.

A further preferred class of compounds of formula (I) because of their herbicidal properties are those having one or more of the following features:

$R^1$ represents a 1-methylethyl, 1-methylcyclopropyl, or a cyclopropyl group;

$R^5$ represents a methyl, ethyl or trifluoromethyl group;

at least one of the groups $R^2$ and $R^4$ represents $-S(O)_mCH_3$, provided that $R^2$ and $R^4$ do not simultaneously represent $-SO_2CH_3$;

q represents two·

A particularly preferred class of compounds of general formula (I) because of their herbicidal properties are those wherein;

$R^1$ represents a cyclopropyl group;

$R^2$ represents a chlorine, bromine, or fluorine atom; or a group selected from methyl, trifluoromethyl and $-S(O)_mCH_3$;

$R^3$ represents $-O(CH_2)_q-OR^5$ ;

$R^4$ represents a chlorine, bromine, or fluorine atom, or a group selected from methyl, trifluoromethyl and $-S(O)_mCH_3$;

at least one of the groups $R^2$ and $R^4$ represents $-S(O)_mCH_3$, provided that $R^2$ and $R^4$ do not simultaneously represent $-SO_2CH_3$; and

q represents two.

Particularly preferred compounds because of their herbicidal activity include the following:

1. 4-[2,4-dibromo-3-(2-methoxyethoxy)benzoyl]-5-cyclopropylisoxazole;
2. 4-[2-bromo-3-(2-methoxyethoxy)-4-methylsulphonylbenzoyl]-5-cyclopropylisoxazole;
3. 4-[2-bromo-3-(2-methoxyethoxy)-4-methylsulphonylbenzoyl]-5-(1-methylcyclopropyl)isoxazole;
4. 4-[2-bromo-3-(2-methoxyethoxy)-4-methylsulphonylbenzoyl]-5-methylisoxazole
5. 4-[2-chloro-3-(2-methoxyethoxy)-4-methylsulphonylbenzoyl]-5-cyclopropylisoxazole;
6. 4-[2-chloro-3-(2-methoxyethoxy)-4-methylsulphonylbenzoyl]-5-(1-methylethyl)isoxazole;
7. 4-[2-chloro-3-(2-methoxyethoxy)-4-methylsulphonylbenzoyl]-5-methylisoxazole;
8. 4-[2-bromo-3-(2-methoxyethoxy)-4-methylsulphenylbenzoyl]-5-cyclopropylisoxazole;
9. 4-[2-bromo-3-(2-methoxyethoxy)-4-methylsulphinylbenzoyl]-5-cyclopropylisoxazole;
10. 4-[4-bromo-3-(2-methoxyethoxy)-2-methylsulphonylbenzoyl]-5-cyclopropylisoxazole;
11. 5-cyclopropyl-4-[2-methylsulphenyl-3-(2-methoxyethoxy)benzoyl]isoxazole;
12. 5-cyclopropyl-4-[2-methylsulphinyl-3-(2-methoxyethoxy)benzoyl]isoxazole; and
13. 5-cyclopropyl-4-[2-methylsulphonyl-3-(2-methoxyethoxy)benzoyl]isoxazole.

The numbers 1 to 13 are assigned to these compounds for reference and identification hereafter.

Compounds of general formula (I) may be prepared by the application or adaptation of known methods (i.e. methods heretofore used or described in the literature), for example as hereinafter described.

In the following description where symbols appearing in formulae are not specifically defined, it is to be understood that they are "as hereinbefore defined" in accordance with the first definition of each symbol in the specification.

It is to be understood that in the descriptions of the following processes the sequences may be performed in different orders; and that suitable protecting groups may be required to achieve the compounds sought.

According to a feature of the present invention compounds of general formula (I) may be prepared by the reaction of a compound of general formula (II):

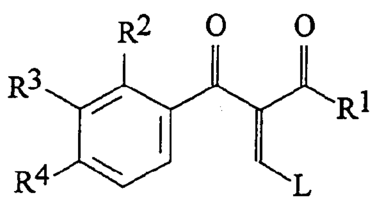

(II)

wherein $R^1$, $R^2$, $R^3$ and $R^4$ are as hereinbefore defined and L is a leaving group, with a salt of hydroxylamine. Hydroxylamine hydrochloride is generally preferred. Generally L is alkoxy, for example ethoxy, or N,N-dialkylamino, for example dimethylamino. The reaction is generally carried out in a solvent such as ethanol or acetonitrile, optionally in the presence of a base or acid acceptor such as triethylamine or sodium acetate.

According to a further feature of the present invention compounds of general formula (I) may be prepared by the reaction of a compound of general formula (III):

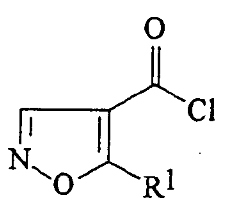

(III)

wherein $R^1$ is as hereinbefore defined, with a compound of general formula (IV):

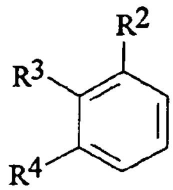

(IV)

wherein $R^2$, $R^3$ and $R^4$ are as hereinbefore defined. The reaction is generally carried out in the presence of a Lewis acid catalyst such as aluminium chloride at a temperature between room temperature and 100°C.

According to a further feature of the present invention compounds of general formula (I) may be prepared by the reaction of a compound of general formula (V):

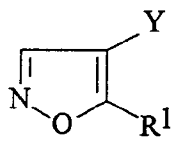

(V)

wherein $R^1$ is as hereinbefore defined and Y represents a carboxy group or a reactive derivative thereof (such as a carboxylic acid chloride or carboxylic ester), or a cyano group, with an appropriate organometallic reagent such as a Grignard reagent or an organolithium reagent. The reaction is generally carried out in an inert solvent such as ether or tetrahydrofuran at a temperature from 0°C to the reflux temperature of the mixture.

Intermediates in the preparation of compounds of general formula (I) may be prepared by the application or adaptation of known methods.

Compounds of general formula (II) may be prepared by the reaction of a compound of general formula (VI):

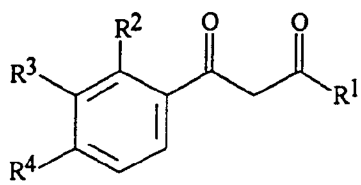

(VI)

wherein $R^1$, $R^2$, $R^3$ and $R^4$ are as hereinbefore defined, with either a trialkyl orthoformate such as triethyl orthoformate or a dimethylformamide dialkylacetal such as dimethylformamide dimethyl acetal. The reaction with triethyl orthoformate is generally carried out in the presence of acetic anhydride at the reflux temperature of the mixture and the reaction with dimethyl formamide dialkyl acetal is carried out optionally in the presence of an inert solvent at a temperature from room temperature to the reflux temperature of the mixture.

The reaction is generally carried out in the presence of an organic base such as triethylamine in an inert solvent such as toluene or dichloromethane at a temperature between -20°C and room temperature.

Compounds of formula (U) may be prepared by the reaction of an acid chloride of formula (VII):

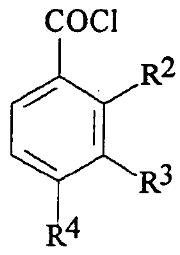

(VII)

wherein $R^2$, $R^3$ and $R^4$ are as hereinbefore defined, with the metal salt of a compound of formula (VIII):

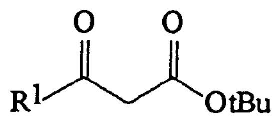

(VIII)

wherein $R^1$ is as hereinbefore defined, to give a compound of formula (IX):

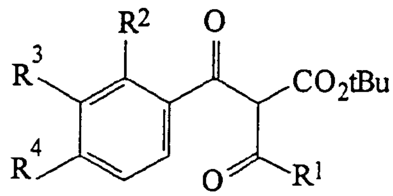

(IX)

wherein $R^1$, $R^2$, $R^3$ and $R^4$ are as hereinbefore defined, which is subsequently decarboxylated to give a compound of formula (VI). Generally the reaction to produce the compound of formula (IX) is performed in a solvent such as a lower alcohol, preferably methanol, in the presence of a metal, preferably magnesium. The decarboxylation is generally performed by refluxing the compound of formula (IX) in the presence of a catalyst, such as paratoluenesulphonic acid, in an inert solvent e.g. toluene.

Intermediates of general formula (III), (IV), (V), (VII) and (VIII) are known or may be prepared by the application or adaptation of known methods.

Those skilled in the art will appreciate that some compounds of general formula (I) may be prepared by the interconversion of other compounds of general formula (I) and such interconversions constitute yet more features of the present invention. Examples of such interconversions are hereafter described.

According to a further feature of the present invention compounds in which m is one or two may be prepared by the oxidation of the sulphur atom of compounds in which m is zero. The oxidation of the sulphur atom is generally carried out using for example 3-chloroperoxybenzoic acid in an inert solvent such as dichloromethane at a temperature from -40°C to room temperature.

The following examples illustrate the preparation of compounds of general formula (I) and the following reference examples illustrate the preparation of intermediates of the invention. In the present specification bp means boiling point; m.p. means melting point. Where the letters NMR appear the characteristics of the proton nuclear magnetic resonance spectrum follow.

<u>Example 1</u>

Sodium acetate (1.65g) was added to a stirred mixture of 3-cyclopropyl-2-ethoxymethylene-1-[2-bromo-3-(2-methoxyethoxy)-4-methylsulphonylphenyl]propan-1,3-dione (8.5g) and hydroxylamine hydrochloride (1.77g) in ethanol. The mixture was stirred at room temperature overnight and then evaporated to dryness.

The residue was dissolved in ethyl acetate and washed with water, dried (anhydrous magnesium sulphate) and filtered. The filtrate was evaporated to dryness. The residue was chromatographed on silica eluted with a mixture of ethyl acetate and cyclohexane (1:5) to give 4-[2-bromo-3-(2-methoxyethoxy)-4-methylsulphonylbenzoyl]-5-cyclopropylisoxazole (compound 2) as a yellow oil (3.3g), NMR ($CDCl_3$), 1.3(2H, m), 1.4(2H,m), 2.6(1H,m), 3.3(3H,s), 3.5(3H,s), 3.9(2H,m), 4.5(2H,m), 7.3(1H,d), 8.1(1H,d), 8.15(1H,s).

By proceeding in a similar manner the following compounds of general formula (I) were prepared.

| Cpd. No. | $R^1$ | $R^2$ | $R^3$ | $R^4$ | mp/NMR |
|---|---|---|---|---|---|
| 1 | Cp | Br | $O(CH_2)_2OMe$ | Br | 1.15(2H,m) 1.3(2H,m) 2.5(1H,m) 3.4(3H,s) 3.75(2H,t) 4.1(2H,t) 6.8(1H,d) 7.5(1H,d) 8.1(1H,s) |
| 3 | 1-Me-Cp | Br | $O(CH_2)_2OMe$ | $SO_2Me$ | 0.9(2H,m) 1.3(2H,m) 1.4(3H,s) 3.2(3H,s) 3.4(3H,s) 3.8(2H,t) 4.4(2H,t) 7.2(1H,d) 8.0(1H,s) 8.1(1H,d) |
| 4 | Me | Br | $O(CH_2)_2OMe$ | $SO_2Me$ | 2.6(3H,s) 3.3(3H,s) 3.45(3H,s) 3.8(2H,t) 4.4(2H,t) 7.2(1H,d) 8.0(1H,d) 8.1(1H,s) |
| 5 | Cp | Cl | $O(CH_2)_2OMe$ | $SO_2Me$ | 81.4-82.6°C |
| 6 | iPr | Cl | $O(CH_2)_2OMe$ | $SO_2Me$ | 84.2-86.2°C |
| 7 | Me | Cl | $O(CH_2)_2OMe$ | $SO_2Me$ | 107.2-107.8°C |
| 8 | Cp | Br | $O(CH_2)_2OMe$ | SMe | 94.4-94.8°C |
| 10 | Cp | $SO_2Me$ | $O(CH_2)_2OMe$ | Br | 104.2-105.2°C |
| 11 | Cp | SMe | $O(CH_2)_2OMe$ | H | 59-62°C |

Note: Cp = cyclopropyl

Example 2

3-Chloroperoxybenzoic acid (50-60%) (1.3g) was added to a solution of 5-cyclopropyl-4-[2-bromo-3-(2-methoxyethoxy)-4-methylsulphenyl benzoyl]isoxazole (1.9g) in dichloromethane whilst maintaining the temperature at around -15°C. The mixture was stirred at -15°C for 1 hour and at room temperature for 1 hour. The solution was diluted with dichloromethane and washed with sodium bisulphite solution (2M), followed by water. The organic layer was dried (anhydrous magnesium sulphate) and filtered. The filtrate was evaporated to dryness. The residue was purified by dry column flash chromatography eluted with a mixture of ethyl acetate and cyclohexane (1:5) yielding 5-cyclopropyl-4-[2-bromo-3-(2-methoxyethoxy)-4-methylsulphinylbenzoyl]isoxazole (Compound 9) (1.52g) as a white solid, mp 86.2-87.0°C.

By proceeding in a similar manner the following compounds were prepared:

5-cyclopropyl-4-[2-methylsulphinyl-3-(2-methoxyethoxy)]benzoylisoxazole (compound 12), m.p. 82 to 84°C;

5-cyclopropyl-4-[2-methylsulphonyl-3-(2-methoxyethoxy)]benzoylisoxazole (compound 13), m.p. 111 to 114°C.

Reference Example 1

A mixture of 3-cyclopropyl-1-[2-bromo-3-(2-methoxyethoxy)-4-methylsulphonylphenyl]propan-1,3-dione (7.5g) and triethylorthoformate (10.3g) in acetic anhydride was stirred and heated at reflux for 3 hours. It was evaporated to dryness and the residue was treated with xylene and re-evaporated to give crude 3-cyclopropyl-1-[2-bromo-3-(2-methoxyethoxy)-4-methylsulphonylphenyl]-2-ethoxymethylenepropan-1,3-dione (8.5g) as a dark brown oil which was not purified further.

By proceeding in a similar manner the following compounds were prepared (but not purified) from the appropriately substituted starting materials.

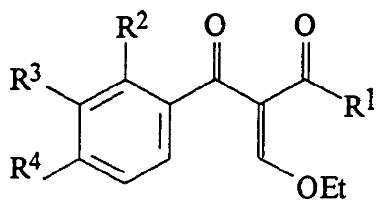

| $R^1$ | $R^2$ | $R^3$ | $R^4$ |
|---|---|---|---|
| Cp | Br | $O(CH_2)_2OMe$ | Br |
| 1-Me-Cp | Br | $O(CH_2)_2OMe$ | $SO_2Me$ |
| Me | Br | $O(CH_2)_2OMe$ | $SO_2Me$ |
| Cp | Cl | $O(CH_2)_2OMe$ | $SO_2Me$ |
| iPr | Cl | $O(CH_2)_2OMe$ | $SO_2Me$ |
| Cp | Br | $O(CH_2)_2OMe$ | SMe |
| Cp | $SO_2Me$ | $O(CH_2)_2OMe$ | Br |
| Cp | SMe | $O(CH_2)_2OMe$ | H |

Note: Cp = cyclopropyl

Reference Example 2

A mixture of crude t-butyl 2-[2-bromo-3-(2-methoxyethoxy)-4-methylsulphonylbenzoyl]-3-cyclopropyl-3-oxopropanoate (10.2g) and 4-toluenesulphonic acid (2g) in dry toluene was stirred and heated at reflux for 2 hours. The cooled mixture was washed with water, dried (anhydrous magnesium sulphate) and filtered. The

filtrate was evaporated to dryness to give 3-cyclopropyl-1-[2-bromo-3-(2-methoxyethoxy)-4-methylsulphonylphenyl]propan-1,3-dione (7.5g) as a brown oil,NMR($CDCl_3$) 0.9(4H,m), 1.5(1H,m), 3.1(3H,s), 3.3(3H,s), 3.6(2H,t), 4.2(2H,t), 5.7(1H,s), 7.15(1H,d), 7.8(1H,d).

By proceeding in a similar manner the following compounds were prepared from the appropriately substituted starting materials.

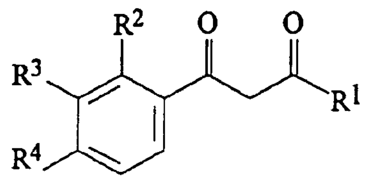

| $R^1$ | $R^2$ | $R^3$ | $R^4$ | NMR/m.p. |
|---|---|---|---|---|
| Cp | Br | $O(CH_2)_2OMe$ | Br | 1.5(4H,m) 2.1(1H,m) 3.9(3H,s) 4.2(2H,t) 4.6(2H,t) 6.3(1H,s) 7.4(1H,d) 7.9(1H,d) |
| 1-Me-Cp | Br | $O(CH_2)_2OMe$ | $SO_2Me$ | 1.2(2H,m) 1.5(2H,m) 1.7(3H,s) 3.6(3H,s) 3.8(3H,s) 4.15(2H,t) 4.7(2H,t) 6.2(1H,s) 7.65(1H,d) 8.3(1H,d) |
| Me | Br | $O(CH_2)_2OMe$ | $SO_2Me$ | 2.4(3H,s) 3.5(3H,s) 3.7(3H,s) 4.0(2H,t) 4.6(2H,t) 6.1(1H,s) 7.55(1H,d) 8.15(1H,d) |
| Cp | Cl | $O(CH_2)_2OMe$ | $SO_2Me$ | 1.1(4H,m) 1.7(1H,m) 3.3(3H,s) 3.5(3H,s) 3.8(2H,t) 4.3(2H,t) 5.9(1H,s) 7.3(1H,d) 7.7(1H,d) |
| iPr | Cl | $O(CH_2)_2OMe$ | $SO_2Me$ | 1.1(6H,d) 2.4(1H,m) 3.1(3H,s) 3.3(3H,s) 3.6(2H,t) 4.2(2H,t) 5.7(1H,s) 7.1(1H,d) 7.7(1H,d) |
| Me | Cl | $O(CH_2)_2OMe$ | $SO_2Me$ | 2.4(3H,s) 3.5(3H,s) 3.7(3H,s) 4.0(2H,t) 4.6(2H,t) 6.0(1H,s) 7.4(1H,d) 8.0(1H,d) |
| Cp | Br | $O(CH_2)_2OMe$ | SMe | 1.2(4H,m) 1.8(1H,m) 2.5(3H,s) 3.5(3H,s) 3.8(2H,t) 4.2(2H,t) 6.0(1H,s) 7.1(2H,m) |
| Cp | $SO_2Me$ | $O(CH_2)_2OMe$ | Br | 1.0(4H,m) 2.0(1H,m) 3.3(3H,s) 3.5(3H,s) 3.9(2H,t) 4.4(2H,t) 5.75(1H,s) 7.0(1H,d) 7.7(1H,d) |
| Cp | SMe | $O(CH_2)_2OMe$ | H | - |

Note: Cp = cyclopropyl

Reference Example 3

Carbon tetrachloride (1 ml) was added to a stirred mixture of t-butyl-3-cyclopropyl-3-oxopropionate (4.5g) and magnesium (0.68g) in methanol, causing a vigorous reaction. The mixture was stirred for 0.25 hours and evaporated to dryness. The residue was dissolved in acetonitrile and a solution of 2-bromo-3-(2-methoxyethoxy)-4-methylsulphonylbenzoyl chloride in acetonitrile (50ml) was added and the resultant mixture stirred at room temperature for 2 hours then left to stand overnight. The acetonitrile was removed under reduced pressure and the residue was suspended in toluene. The toluene suspension was washed with 2M HCl, followed by water. The resulting organic solution was dried (anhydrous magnesium sulphate) and filtered. The filtrate was evaporated to dryness to give crude tert-butyl 2-[2-bromo-3-(2-methoxyethoxy)-4-methylsulphonylbenzoyl]-3-cyclopropyl-3-oxopropionate (10.27g) as a crude brown oil.

By proceeding in a similar manner the following compounds were prepared:

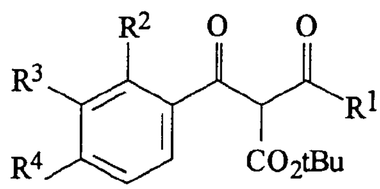

| $R^1$ | $R^2$ | $R^3$ | $R^4$ | Reaction Solvent |
|---|---|---|---|---|
| Cp | Br | $O(CH_2)_2OMe$ | Br | $CH_3CN$ |
| 1-Me-Cp | Br | $O(CH_2)_2OMe$ | $SO_2Me$ | Toluene |
| Me | Br | $O(CH_2)_2OMe$ | $SO_2Me$ | Toluene |
| Cp | Cl | $O(CH_2)_2OMe$ | $SO_2Me$ | $CH_3CN$ |
| iPr | Cl | $O(CH_2)_2OMe$ | $SO_2Me$ | $CH_3CN$ |
| Me | Cl | $O(CH_2)_2OMe$ | $SO_2Me$ | $CH_3CN$ |
| Cp | Br | $O(CH_2)_2OMe$ | SMe | $CH_3CN$ |
| Cp | $SO_2Me$ | $O(CH_2)_2OMe$ | Br | $CH_3CN$ |
| Cp | SMe | $O(CH_2)_2OMe$ | H | $CH_3CN$ |

Benzoyl chlorides were prepared by heating the appropriately substituted benzoic acids at reflux with thionyl chloride for 3 hours.

Reference Example 4

Ethyl 2-bromo-3-(2-methoxyethoxy)-4-methylsulphenylbenzoate (24.3g) was added to a solution of potassium hydroxide (9.1g) in water (25ml) and industrial methylated spirits (300ml). The resultant mixture was heated at reflux for 4 hours and then allowed to cool to room temperature overnight. The mixture was evaporated and the residue dissolved in water and washed with ether. The aqueous layer was acidified to pH 1 with 2M HCl and extracted with ethyl acetate. The ethyl acetate layer was washed with water, dried (anhydrous magnesium sulphate) and filtered. The filtrate was evaporated to dryness yielding 2-bromo-3-(2-methoxyethoxy)-4-methylsulphenyl benzoic acid (19.4g) as a white solid, NMR ($CDCl_3$) 2.2(3H,s), 3.25(3H,s), 3.6(2H,t), 3.8(2H,t), 7.0(2H,m).

By proceeding in a similar manner 4-bromo-3-(2-methoxyethoxy)-2-methylsulphonylbenzoic acid was prepared as a white solid, m.p. 156-157.8°C starting from ethyl 4-bromo-3-(2-methoxyethoxy)-2-methylsulphonylbenzoate.

Reference Example 5

Ethyl 4-bromo-3-(2-methoxyethoxy)-2-methylsulphenylbenzoate (5.0g) was stirred in acetic acid (8.0ml) and acetic anhydride (2.0ml) at 0°C. To the mixture was added hydrogen peroxide (11 ml). The mixture was stirred at 0°C for 1 hour and then overnight at room temperature. The mixture was then heated at 85°C for 1 hour. After cooling to room temperature, the mixture was diluted with ethyl acetate and washed with water, followed by ferrous sulphate, followed by water. The organic extract was dried (anhydrous magnesium sulphate) and filtered. The filtrate was evaporated to dryness yielding ethyl 4-bromo-3-(2-methoxyethoxy)-2-methylsulphonylbenzoate (4.7g) NMR ($CDCl_3$) 1.8(3H,t), 3.4(3H,s), 3.8(3H,s), 4.15(2H,t), 4.3(2H,q), 4.6(2H,t), 7.15(H,d), 7.85(1H,d).

Reference Example 6

Ethyl 2,4-dibromo-3-(2-methoxyethoxy)benzoate(32.2g) was stirred in DMF (80ml) with potassium carbonate (36g). To the resultant suspension was added a solution of methane thiol (13ml) in DMF(20ml). The resultant mixture was stirred overnight at room temperature. It was then diluted with ether, and the mixture washed with water, dried (anhydrous magnesium sulphate) and filtered. The filtrate was evaporated to dryness. The residue was purified by column chromatography on silica eluting with ethyl acetate and cyclohexane (1:20) which yielded ethyl 4-bromo-3-(2-methoxyethoxy)-2-methylsulphenylbenzoate (3.5g) as an oil which was not further characterised.

Reference Example 7

2-[3-(2-Methoxyethoxy)-2-methylsulphenylphenyl]-4,4-dimethyl-2-oxazoline (48.7 g) was added to a 3M solution of hydrochloric acid (700 ml) at 90°C. The mixture was stirred at 90°C for 3 hours, cooled to room temperature and extracted with ethyl acetate. The organic extract was washed with water, dried (anhydrous magnesium sulphate) and filtered. The filtrate was evaporated to dryness yielding 3-(2-methoxyethoxy)-2-methylsulphenylbenzoic acid as a golden oil (40.9 g), NMR $CDCl_3$ 2.5 (3H,s), 3.5(3H,s), 3.8(2H,m), 4.3(2H,t), 7.1(1H,m), 7.4(1H,m), 7.6(1H,m).

Reference Example 8

A solution of 2-[3-(2-methoxyethoxy)phenyl]-4,4-dimethyl-2-oxazoline (63.4 g) in tetrahydrofuran was cooled to -40°C under an inert atmosphere. n-Butyl lithium (122 ml of 2.5m) was added to the solution. The resultant mixture was stirred at -15°C for one hour and then re-cooled to -40°C. Dimethyl disulphide (52.7 g) was added and the reaction mixture was then allowed to warm to room temperature and stirred for 1 hour. Saturated ammnonium chloride solution was added and the mixture extracted with ether. The organic extract was washed with water, dried (anhydrous magnesium sulphate) and filtered. The filtrate was evaporated to dryness and the residue recrystallised from hexane yielding 2-[3-(2-methoxyethoxy)-2-methylsulphenylphenyl]-4,4-dimethyl-2-oxazoline (56.5 g) as a powder, m.p. 44.4-46.4°C.

Reference Example 9

N-(2-Hydroxy-1,1-dimethylethyl)-3-(2-methoxyethoxy)benzamide was stirred at 0°C. Thionyl chloride (90 ml) was added over one hour. The resulting solution was stirred at 15°C for a further 1.5 hours and dry diethyl ether was added. The excess solvents were removed under vaccum and the residue was suspended in diethyl ether, washed with 2M sodium hydroxide solution and the organic phase separated. The organic extract was washed with water, dried (anhydrous magnesium sulphate) and filtered. The filtrate was evaporated to dryness leaving a residue which was distilled under reduced pressure to give 2-[3-(2-methoxyethoxy)phenyl]-4,4-dimethyl-2-oxazoline (66 g) b.p. 164-168°C at 1.5mm Hg.

Reference Example 10

2-Amino-2-methylpropan-1-ol (57.6g) was dissolved in dichloromethane and cooled to 0°C. A solution of 3-(2-methoxyethoxy) benzoyl chloride (67.8g) in dichloromethane was added and the solution was stirred overnight at room temperature during which time a precipitate formed. The mixture was filtered and the filtrate evaporated to dryness yielding N-(2-hydroxy-1,1-dimethylethyl)-3-(2-methoxyethoxy)benzamide (95.9g) as a syrup, NMR: $CDCl_3$ 1.4(6H,s), 3.5(3H,s), 3.65(2H,s), 3.7(2H,t), 4.2(2H,t), 5.4(1H,s), 6.3(1H,s), 7.0(1H,m),

7.3(2H,m).

Reference Example 11

3-(2-methoxyethoxy)benzoic acid (72.9g) and thionyl chloride (119g) were heated together at reflux for 1.5 hours and then allowed to cool overnight. The thionyl chloride was evaporated under reduced pressure. The residue was distilled under reduced pressure to give 3-(2-methoxyethoxy)benzoyl chloride (67.7g), b.p. 130°C/0.4 mm Hg.

Reference Example 12

2-Methoxyethyl 3-(2-methoxyethoxy)benzoate (133 g) was dissolved in industrial methylated spirits and 2M sodium hydroxide solution and heated to reflux for 1.5 hours. The mixture was allowed to cool and ice was added. The resultant mixture was washed with ether, the aqueous layer was acidified to pH 1 with hydrochloric acid and the resultant mixture was filtered. The residue was washed with water and air dried. The residue was recrystallised from ether and petroleum ether (b.p. 40-65°C) yielding 3-(2-methoxyethoxy)benzoic acid (86 g) as a white solid m.p. 79.6-81.8°C.

Reference Example 13

3-Hydroxybenzoic acid (73 g) was dissolved in dimethyl formamide and potassium carbonate (160 g) 2-chloroethyl methyl ether (200 g) and potassium iodide (8.8 g) were added. The mixture was heated at 72°C for 42 hours. After cooling the mixture was poured onto ice and extracted with ether. The organic extract was washed with 2M sodium hydroxide solution, water, brine and was dried (anhydrous magnesium sulphate) and filtered. The filtrate was evaporated to dryness to yield 2-methoxyethyl 3-(2-methoxyethoxy)benzoate (135.5g) which was not purified further.

According to a feature of the present invention, there is provided a method for controlling the growth of weeds (i.e. undesired vegetation) at a locus which comprises applying to the locus a herbicidally effective amount of at least one isoxazole derivative of general formula (I). For this purpose, the isoxazole derivatives are normally used in the form of herbicidal compositions (i.e. in association with compatible diluents or carriers and/or surface active agents suitable for use in herbicidal compositions), for example as hereinafter described. The compounds of general formula (I) show herbicidal activity against dicotyledonous (i.e. broad-leafed) and monocotyledonous (i.e. grass) weeds by pre- and/or post-emergence application. By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula (I) may be used to control the growth of:

broad-leafed weeds, for example, Abutilon theophrasti, Amaranthus retroflexus, Bidens pilosa, Chenopodium album, Galium aparine, Ipomoea spp. e.g. Ipomoea purpurea, Sesbania exaltata, Sinapis arvensis, Solanum nigrum and Xanthium strumarium, and

grass weeds, for example Alopecurus myosuroides, Avena fatua, Digitaria sanguinalis, Echinochloa crus-galli, Sorghum bicolor, Eleusine indica and Setaria spp, e.g. Setaria faberii or Setaria viridis, and

sedges, for example, Cyperus esculentus.

The amounts of compounds of general formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.01kg and 4kg of active material per hectare give good results. However, it is to be understood that higher or lower application rates may be used, depending upon the particular problem of weed control encountered.

The compounds of general formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates

between 0.01kg and 4.0kg, and preferably between 0.01kg and 2.0kg, of active material per hectare are particularly suitable.

The compounds of general formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non- directional fashion (e.g. by directional or non-directional spraying) to the weeds or to the soil in which they are expected to appear, before or after planting of the trees or plantations at application rates between 0.25kg and 5.0kg, and preferably between 0.5kg and 4.0kg of active material per hectare.

The compounds of general formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1.0kg and 20.0kg, and preferably between 5.0 and 10.0kg, of active material per hectare are particularly suitable for this purpose. When used to control the growth of weeds by pre-emergence application, the compounds of general formula (I) may be incorporated into the soil in which the weeds are expected to emerge. It will be appreciated that when the compounds of general formula (I) are used to control the growth of weeds by post-emergence application, i.e. by application to the aerial or exposed portions of emerged weeds, the compounds of general formula (I) will also normally come into contact with the soil and may also then exercise a pre-emergence control on later-germinating weeds in the soil.

Where especially prolonged weed control is required, the application of the compounds of general formula (I) may be repeated if required.

According to a further feature of the present invention there are provided compositions suitable for herbicidal use comprising one or more of the isoxazole derivatives of general formula (I), in association with, and preferably homogeneously dispersed in, one or more compatible agriculturally- acceptable diluents or carriers and/or surface active agents [i.e. diluents or carriers and/or surface active agents of the type generally accepted in the art as being suitable for use in herbicidal compositions and which are compatible with compounds of general formula (I)]. The term "homogeneously dispersed" is used to include compositions in which the compounds of general formula (I) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula (I).

The herbicidal compositions may contain both a diluent or carrier and surface-active (e.g. wetting, dispersing, or emulsifying) agent. Surface-active agents which may be present in herbicidal compositions of the present invention may be of the ionic or nonionic types, for example sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, talc, calcined magnesia, kieselguhr, tricalcium phosphate, powdered cork, absorbent carbon black and clays such as kaolin and bentonite. The solid compositions (which may take the form of dusts, granules or wettable powders) are preferably prepared by grinding the compounds of general formula (I) with solid diluents or by impregnating the solid diluents or carriers with solutions of the compounds of general formula (I) in volatile solvents, evaporating the solvents and,

if necessary, grinding the products so as to obtain powders. Granular formulations may be prepared by absorbing the compounds of general formula (I) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of general formula (I) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Unless otherwise specified, the following percentages are by weight. Preferred herbicidal compositions according to the present invention are

aqueous suspension concentrates which comprise from 10 to 70% of one or more compounds of general formula (I), from 2 to 10% of surface-active agent, from 0.1 to 5% of thickener and from 15 to 87.9% of water;

wettable powders which comprise from 10 to 90% of one or more compounds of general formula (I), from 2 to 10% of surface-active agent and from 8 to 88% of solid diluent or carrier;

water soluble or water dispersible powders which comprise from 10 to 90% of one or more compounds of general formula (I), from 2 to 40% of sodium carbonate and from 0 to 88% of solid diluent;

liquid water soluble concentrates which comprise from 5 to 50%, e.g. 10 to 30%, of one or more compounds of general formula (I), from 5 to 25% of surface-active agent and from 25 to 90%, e.g. 45 to 85 %, of water miscible solvent, e.g. dimethylformamide, or a mixture of water-miscible solvent and water;

liquid emulsifiable suspension concentrates which comprise from 10 to 70% of one or more compounds of general formula (I), from 5 to 15% of surface-active agent, from 0.1 to 5% of thickener and from 10 to 84.9% of organic solvent;

granules which comprise from 1 to 90%, e.g. 2 to 10% of one or more compounds of general formula (I), from 0.5 to 7%, e.g. 0.5 to 2%, of surface-active agent and from 3 to 98.5%, e.g. 88 to 97.5%, of granular carrier and

emulsifiable concentrates which comprise 0.05 to 90%, and preferably from 1 to 60% of one or more compounds of general formula (I), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula (I) in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled for example alachlor [2-chloro-2,6'-diethyl-N-(methoxy-methyl)acetanilide], atrazine [2-chloro-4-ethylamino-6-isopropylamino-1,3,5-triazine], bromoxynil [3,5-dibromo-4-hydroxybenzonitrile], chlortoluron [N'-(3-chloro-4-methylphenyl)-N,N-dimethylurea], cyanazine [2-chloro-4-(1-cyano-1-methylethylamino)-6-ethylamino-1,3,5-triazine], 2,4-D [2,4-dichlorophenoxy-acetic acid], dicamba [3,6-dichloro-2-methoxybenzoic acid], difenzoquat [1,2-dimethyl-3-,5-diphenyl-pyrazolium salts], flampropmethyl [methyl N-2-(N-benzoyl-3-chloro-4-fluoroanilino)-propionate], fluometuron [N'-(3-trifluoro-methylphenyl)-N,N-dimethylurea], isoproturon [N'-(4-isopropylphenyl)-N,N-dimethylurea], insecticides, e.g. synthetic pyrethroids, e.g. permethrin and cypermethrin, and fungicides, e.g. carbamates, e.g. methyl N-(1-butyl-carbamoyl-benzimidazol-2-yl)carbamate, and triazoles e.g. 1-(4-chlorophenoxy)-3,3-dimethyl-1-(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the isoxazole derivatives of general formula (I) or, as is preferred, a herbicidal composition as hereinbefore described, and preferably a herbicidal concentrate which must be diluted before use, comprising at least one of the isoxazole derivatives of general formula (I) within a container for the aforesaid derivative or derivatives of general formula (I), or a said herbicidal composition, and instructions physically associated with the aforesaid container setting out the manner in which the aforesaid derivative or derivatives of general formula (I) or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the isoxazole derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01kg and 20kg of active material per hectare in the manner and for the purposes hereinbefore described.

The following Examples illustrate herbicidal compositions according to the present invention:

## EXAMPLE C1

A soluble concentrate is formed from :

| | |
|---|---|
| Active ingredient (compound 1) | 20% w/v |
| Potassium hydroxide solution 33% w/v | 10% v/v |
| Tetrahydrofurfuryl alcohol (THFA) | 10% v/v |
| Water | to 100 volumes. |

by stirring THFA active ingredient (compound 1) and 90% volume of water and slowly adding the potassium hydroxide solution until a steady pH 7-8 is obtained then making up to volume with water.

Similar soluble concentrates may be prepared as described above by replacing the isoxazole (compound 1) with other compounds of general formula (I).

## EXAMPLE C2

A wettable powder is formed from :

| | |
|---|---|
| Active ingredient (compound 1) | 50% w/v |
| Sodium dodecylbenzene sulphonate | 3% w/w |
| Sodium lignosulphate | 5% w/w |
| Sodium formaldehyde alkylnaphthalene sulphonate | 2% w/w |
| Microfine silicon dioxide | 3% w/w and |
| China clay | 37% w/w |

by blending the above ingredients together and grinding the mixture in an air jet mill.

Similar wettable powders may be prepared as described above by replacing the isoxazole (compound 1) with other compounds of general formula (I).

**EXAMPLE C3**

A water soluble powder is formed from :

| | |
|---|---|
| Active ingredient (compound 1) | 50% w/w |
| Sodium dodecylbenzenesulphonate | 1% w/w |
| Microfine silicon dioxide | 2% w/w |
| Sodium bicarbonate | 47% w/w |

by mixing the above ingredients and grinding the above mixture in a hammer mill.

Similar water soluble powders may be prepared as described above by replacing the isoxazole (compound 1) with other compounds of general formula (I).

The compounds of the invention have been used in herbicidal applications according to the following procedures.

**METHOD OF USE OF HERBICIDAL COMPOUNDS:**

a) General

Appropriate quantities of the compounds used to treat the plants were dissolved in acetone to give solutions equivalent to application rates of up to 4000g test compound per hectare (g/ha). These solutions were applied from a standard laboratory herbicide sprayer delivering the equivalent of 290 litres of spray quid per hectare.

b) Weed control : Pre-emergence

The seeds were sown in 70 mm square, 75 mm deep plastic pots in non-sterile soil . The quantities of seed per pot were as follows:-

| Weed species | Approx number of seeds/pot |
|---|---|
| 1) Broad-leafed weeds | |
| Abutilon theophrasti | 10 |
| Amaranthus retroflexus | 20 |
| Galium aparine | 10 |
| Ipomoea purpurea | 10 |
| Sinapis arvensis | 15 |
| Xanthium strumarium | 2. |
| 2) Grass weeds | |
| Alopecurus myosuroides | 15 |
| Avena fatua | 10 |
| Echinochloa crus-galli | 15 |
| Setaria viridis | 20. |
| 3) Sedges | |
| Cyperus esculentus | 3. |
| Crop | |
| 1) Broad-leafed | |
| Cotton | 3 |
| Soya | 3. |
| 2) Grass | |
| Maize | 2 |
| Rice | 6 |
| Wheat | 6. |

The compounds of the invention were applied to the soil surface, containing the seeds, as described in (a). A single pot of each crop and each weed was allocated to each treatment, with unsprayed controls and controls sprayed with acetone alone.

After treatment the pots were placed on capillary matting kept in a glass house, and watered overhead . Visual assessment of crop damage was made 20-24 days after spraying. The results were expressed as the percentage reduction in growth or damage to the crop or weeds, in comparison with the plants in the control pots.

c) Weed control : Post-emergence

The weeds and crops were sown directly into John Innes potting compost in 75 mm deep, 70 mm square pots except for Amaranthus which was pricked out at the seedling stage and transferred to the pots one week before spraying. The plants were then grown in the greenhouse until ready for spraying with the compounds used to treat the plants. The number of plants per pot were as follows :-

| 1) Broad leafed weeds | | |
|---|---|---|
| Weed species | Number of plants per pot | Growth stage |
| Abutilon theophrasti | 3 | 1-2 leaves |
| Amaranthus retroflexus | 4 | 1-2 leaves |
| Galium aparine | 3 | 1st whorl |
| Ipomoea purpurea | 3 | 1-2 leaves |
| Sinapis arvensis | 4 | 2 leaves |
| Xanthium strumarium | 1 | 2-3 leaves. |

| 2) Grass weeds | | |
|---|---|---|
| Weed species | Number of plants per pot | Growth stage |
| Alopecurus myosuroides | 8-12 | 1-2 leaves |
| Avena fatua | 12-18 | 1-2 leaves |
| Echinochloa crus-galli | 4 | 2-3 leaves |
| Setaria viridis | 15-25 | 1-2 leaves. |

| 3) Sedges | | |
|---|---|---|
| Weed species | Number of plants per pot | Growth stage |
| Cyperus esculentus | 3 | 3 leaves. |

| 1) Broad leafed | | |
|---|---|---|
| Crops | Number of plants per pot | Growth stage |
| Cotton | 2 | 1 leaf |
| Soya | 2 | 2 leaves. |

| 2) Grass | | |
|---|---|---|
| Crops | Number of plants per pot | Growth stage |
| Maize | 2 | 2-3 leaves |
| Rice | 4 | 2-3 leaves |
| Wheat | 5 | 2-3 leaves. |

The compounds used to treat the plants were applied to the plants as described in (a). A single pot of each crop and weed species was allocated to each treatment, with unsprayed controls and controls sprayed with acetone alone.

After treatment the pots were placed on capillary matting in a glass house, and watered overhead once after 24 hours and then by controlled sub-irrigation. Visual assessment of crop damage and weed control was made 20-24 days after spraying. The results were expressed as the percentage reduction in growth or damage to the crop or weeds, in comparison with the plants in the control pots.

The compounds of the invention, used at 4kg/ha or less, have shown an excellent level of herbicidal activity together with crop tolerance on the weeds used in the foregoing experiments.

When applied pre- or post- emregence at 1000g/ha compounds 1 to 13 gave at least 90% reduction in the growth of at least one weed species.

## Claims

1. A 4-benzoylisoxazole derivative of general formula (I):

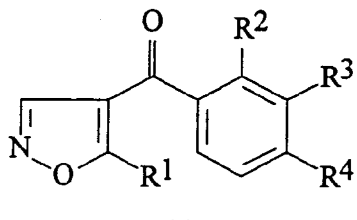

(I)

wherein:

$R^1$ represents a straight- or branched- chain alkyl group containing from one to four carbon atoms or a three or four membered cycloalkyl group which is optionally substituted by a straight- or branched-chain alkyl group containing up to four carbon atoms;

$R^2$ represents a halogen atom, or a group selected from $R^5$, $-OR^5$, $-S(O)_mR^5$, $-O(CH_2)_q-OR^5$, $-CO_2R^5$ and nitro;

$R^3$ represents a halogen atom, or a group selected from $R^5$, $-OR^5$, $-S(O)_mR^5$ and $-O(CH_2)_q-OR^5$;

$R^4$ represents a hydrogen or halogen atom, or a group selected from $R^5$, $-OR^5$, $-S(O)_mR^5$, $-O(CH_2)q-OR^5$ and nitro;

with the proviso that at least one of the groups $R^2$, $R^3$ and $R^4$ represents $-O(CH_2)_q-OR^5$;

$R^5$ represents a straight- or branched- chain alkyl group containing from one to four carbon atoms optionally substituted by one or more halogen atoms;

m represents zero, one or two; and

q represents an integer from one to three.

2. A compound according to claim 1 wherein $R^3$ represents $-O(CH_2)_q-OR^5$.

3. A compound according to claim 1 or 2 wherein:

$R^2$ represents a halogen atom or a group selected from $R^5$, $-OR^5$, $-S(O)_mR^5$, $-CO_2R^5$ and nitro;

$R^3$ represents $-O(CH_2)_q-OR^5$;

$R^4$ represents a hydrogen or halogen atom, or a group selected from $R^5$, $-OR^5$, $-S(O)_mR^5$ and nitro; and

q represents two or three.

4. A compound according to claim 1, 2 or 3 having one or more of the following features:

$R^1$ represents a methyl, ethyl, 1-methylethyl, cyclopropyl or 1-methylcyclopropyl group;

$R^2$ represents a chlorine, bromine or fluorine atom, or a group selected from $R^5$ and $-S(O)_mR^5$;

$R^4$ represents a chlorine, bromine or fluorine atom, or a group selected from $R^5$ and $-S(O)_mR^5$;

$R^5$ represents a straight- or branched- chain alkyl group containing from one to four carbon atoms optionally substituted by one or more halogen atoms.

5. A compound according to any one of the preceding claims having one or more of the following features:

$R^1$ represents a 1-methylethyl, 1-methylcyclopropyl, or a cyclopropyl group;

$R^5$ represents a methyl, ethyl or trifluoromethyl group;

at least one of the groups $R^2$ and $R^4$ represents $-S(O)_mCH_3$, provided that $R^2$ and $R^4$ do not simultaneously represent $-SO_2CH_3$;

q represents two·

6. A compound according to any one of the preceding claims wherein:

$R^1$ represents a cyclopropyl group;

$R^2$ represents a chlorine, bromine, or fluorine atom; or a group selected from methyl, trifluoromethyl and $-S(O)_mCH_3$;

$R^3$ represents $-O(CH_2)q-OR^5$;

$R^4$ represents a chlorine, bromine, or fluorine atom, or a group selected from methyl, trifluoromethyl and $-S(O)_mCH_3$;

at least one of the groups $R^2$ and $R^4$ represents $-S(O)_mCH_3$, provided that $R^2$ and $R^4$ do not simultaneously represent $-SO_2CH_3$;

q represents two.

7. A compound according to claim 1 or 2 which is
4-[2,4-dibromo-3-(2-methoxyethoxy)benzoyl]-5-cyclopropylisoxazole;
4-[2-bromo-3-(2-methoxyethoxy)-4-methylsulphonylbenzoyl]-5-cyclopropylisoxazole;
4-[2-bromo-3-(2-methoxyethoxy)-4-methylsulphonylbenzoyl]-5-(1-methylcyclopropyl)isoxazole;
4-[2-bromo-3-(2-methoxyethoxy)-4-methylsulphonylbenzoyl]-5-methylisoxazole
4-[2-chloro-3-(2-methoxyethoxy)-4-methylsulphonylbenzoyl]-5-cyclopropylisoxazole;
4-[2-chloro-3-(2-methoxyethoxy)-4-methylsulphonylbenzoyl]-5-(1-methylethyl)isoxazole;
4-[2-chloro-3-(2-methoxyethoxy)-4-methylsulphonylbenzoyl]-5-methylisoxazole;
4-[2-bromo-3-(2-methoxyethoxy)-4-methylsulphenylbenzoyl]-5-cyclopropylisoxazole;
4-[2-bromo-3-(2-methoxyethoxy)-4-methylsulphinylbenzoyl]-5-cyclopropylisoxazole;
4-[4-bromo-3-(2-methoxyethoxy)-2-methylsulphonylbenzoyl]-5-cyclopropylisoxazole;
5-cyclopropyl-4-[2-methylsulphenyl-3-(2-methoxyethoxy)benzoyl]isoxazole;
5-cyclopropyl-4-[2-methylsulphinyl-3-(2-methoxyethoxy)benzoyl]isoxazole; or
5-cyclopropyl-4-[2-methylsulphonyl-3-(2-methoxyethoxy)benzoyl]isoxazole.

8. A process for the preparation of a compound of general formula (I) as defined in claim 1 which comprises:
(a) the reaction of a compound of general formula (II):

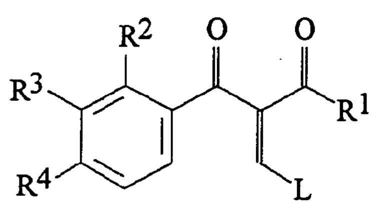
(II)

wherein $R^1$, $R^2$, $R^3$ and $R^4$ are as defined in claim 1 and L is a leaving group, with a salt of hydroxylamine;

(b) the reaction of a compound of general formula (III):

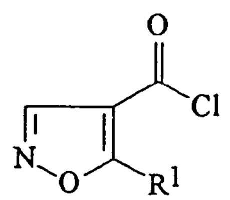
(III)

wherein $R^1$ is as defined in claim 1, with a compound of general formula (IV):

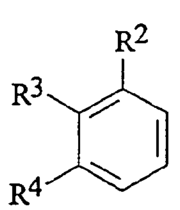
(IV)

wherein $R^2$, $R^3$ and $R^4$ are as defined in claim 1; or

(c) the reaction of a compound of general formula (V):

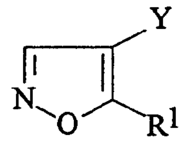

(V)

wherein $R^1$ is defined in claim 1 and Y represents a carboxy group or a reactive derivative thereof, or a cyano group, with an appropriate organometallic reagent.

9. A herbicidal composition which comprises as active ingredient a herbicidally effective amount of a 4-benzoylisoxazole derivative of general formula (I) according to any one of claims 1 to 7, in association with an agriculturally acceptable diluent or carrier and/or surface active agent.

10. A method for controlling the growth of weeds at a locus which comprises applying to the locus a herbicidally effective amount of a 4-benzoylisoxazole derivative of general formula (I) according to any one of claims 1 to 7.

11. A method according to claim 10 in which the locus is an area used, or to be used, for the growing of crops and the compound is applied at an application rate from 0.01 kg to 4.0 kg per hectare.

European Patent Office

# EUROPEAN SEARCH REPORT

Application Number

EP 93 30 0817

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 418 175 (RHONE POULENC AGRICULTURE LTD)<br>* claims 1,7-21 *<br>----- | 1,8-11 | C07D261/08<br>A01N43/80 |
| | | | TECHNICAL FIELDS SEARCHED (Int. Cl.5) |
| | | | C07D |

The present search report has been drawn up for all claims

| Place of search | Date of completion of the search | Examiner |
|---|---|---|
| THE HAGUE | 29 MARCH 1993 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)